# EUROPÄISCHE PATENTSCHRIFT

(11) **EP 0 555 613 B1**
(45) Veröffentlichungstag und Bekanntmachung des Hinweises auf die Patenterteilung: **11.12.1996**
(21) Anmeldenummer: 92810093.2
(22) Anmeldetag: 10.02.1992
(51) Int. Cl.: A61F 2/36, A61F 2/46

(54) **Gerader blattartiger Schaft für eine Endoprothese**
Straight flat stem for an endoprosthesis
Tige droite plate pour une endoprothèse

(43) Veröffentlichungstag der Anmeldung: 18.08.1993
(73) Patentinhaber: Sulzer Orthopädie AG, 6340 Baar (CH)
(72) Erfinder: Ochsner, Peter, Prof.Dr., CH-4402 Renkendorf (CH); Willi, Roland, CH-8413 Neftenbach (CH); Baege, Roland, CH-8404 Winterthur (CH)
(74) Vertreter: Heubeck, Bernhard

(56) Entgegenhaltungen:
- EP-A- 0 131 178
- EP-A- 0 149 527
- EP-A- 0 273 871
- EP-A- 0 366 945
- WO-A-84/03037

## Beschreibung

Die Erfindung betrifft einen geraden blattartigen Schaft für eine Endoprothese, insbesondere eine Hüftgelenksprothese, dessen Blattseiten sich vom distalen, freien Ende aus zunächst symmetrisch zu einer Längsmittelachse konisch erweitern, ehe die laterale Schmalseite parallel zur Längsmittelachse oder auf sie zu verläuft, während die mediale Schmalseite in einem Bogen geführt ist, wobei im proximalen Bereich des Schaftes Führungen vorgesehen sind, in die mindestens ein Klemmkörper einschlagbar ist.

Schäfte der vorstehend geschilderten Art sind beispielsweise bekannt aus der EP-B-0 141 820. Ihre Fixierung erfolgt vorwiegend dadurch, dass der Schaft mit seinen Schmalseiten zwischen medial und lateral "eingespannt" wird, wobei die "Einspannung" möglichst weitgehend über die ganze Schaftlänge erfolgt; die in beide Blattseiten eingetriebenen Klemmkörper dienen dabei als Rotationssicherung.

Aufgabe der vorliegenden Erfindung ist es demgegenüber, eine Fixierung des Schaftes im proximalen Bereich seiner Länge durch eine "Abstützung" in Richtung anterior/posterior zu erreichen und mit der Fixierung gleichzeitig eine Rotationssicherung zu erzielen; darüberhinaus soll dem Operateur bei unter Umständen notwendigen Reoperationen die Möglichkeit gegeben werden, die Fixierung auf relativ einfache Weise zu lösen.

Mit der Erfindung wird diese Aufgabe dadurch gelöst, dass der Klemmkörper ein U-förmiges Blech ist, dessen freie Schenkel zu ihrem Ende hin abgeschrägt sind, und dass ferner parallel zu den Führungen des Schaftes ein blattfederartig federndes Sicherungselement vorgesehen ist, das auf der den Führungen zugewandten Blattfederseite einen nasenartigen Vorsprung trägt, der in eine Vertiefung des eingeschlagenen Klemmkörpers einrastet.

Der als Blech ausgebildete Klemmkörper, dessen U-Form mit dem Vorteil einer vergrösserten proximalen Abstützfläche zu einer H-Form "erweitert" sein kann, deren Querbalken eine Kante zum Einrasten des nasenartigen Vorsprunges bildet, wird bei der Verankerung des Schaftes mit seinen freien Schenkeln voraus auf die Führungen in den Blattseiten aufgeschoben und in den vorher vorbearbeiteten Operationshohlraum eingeschlagen, bis er im proximalen Bereich des Knochens zwischen anterior und posterior verklemmt ist; auf diese Weise bewirkt er gleichzeitig eine Fixierung und eine Sicherung gegen unerwünschte Rotationen. Die Abschrägungen seiner freien Schenkel erleichtern dabei sein Eindringen in den Knochen und dienen einer zusätzlichen vertikalen Abstützung auf der Korticalis. Als Sicherung gegen unbeabsichtigte Lockerungen des Klemmsitzes rastet die Nase des Blattfeder Elementes in eine Vertiefung des Klemmkörpers ein, wobei die federnde Ausbildung dieser Sicherung die geforderte Erleichterung für ein gewolltes Lösen der Fixierung im Falle einer Reoperation bringt.

Eine weitere Vereinfachung ergibt sich beim Einschlagen des Klemmkörpers, wenn die Führungen in den Blattseiten parallel zu ihrer Längsmittelachse verlaufen.

Um eine Anpassung der Schaftverankerung an individuell gegebene "Grössen" eines Patienten mit einer oder wenigen Schaftgrössen erreichen und links- bzw. rechtsseitig gleiche Schäfte und Klemmkörper verwenden zu können, ist es vorteilhaft, wenn der Schaftkörper symmetrisch zu der zu seinen Blattseiten parallel verlaufenden Mittelebene ausgebildet ist, während der senkrecht zu dieser Ebene verlaufende, U-förmige Klemmkörper eine unsymmetrische Form hat, wobei seine Schenkel gleich lang aber zur Anpassung an die Verhältnisse im Knochenhohlraum unterschiedlich breit gestaltet sind, und wobei selbstverständlich jedem Schaft mehrere Klemmkörper mit unterschiedlichen Abmessungen zur Auswahl beigefügt werden können.

Der blattartige Schaft wird zunächst in den Knochenhohlraum eingeschlagen. Zum Vorkonditionieren des Knochenhohlraums anterior und posterior im oberen Schaftbereich wird eine Einschlagschablone, welche die Kontur der Klemmkörper besitzt und welche im tragenden Bereich gegen das Knochengewebe jedoch eine geringere Wandstärke aufweist, geführt vom blattartigen Schaft eingeschlagen, um aufgrund der Eindringtiefe den Klemmkörper zu bestimmen, dessen Kontur die passende Lage in Richtung der Längsmittelachse aufweist, bei der das Sicherungselement am Klemmkörper einrastet und bei der eine Klemmung gegen das Knochengewebe stattfindet. Nach dem Ziehen der Einschlagschablone wird der so bestimmte Klemmkörper eingeschlagen bis eine Verklinkung mit dem Sicherungselement stattgefunden hat.

Bevorzugte Werkstoffe sowohl für den Schaft als auch für den Klemmkörper und die Einschlagschablone sind Metalle oder Metallegierungen, insbesondere Titan oder Titanlegierungen.

Die Erfindung betrifft ferner einen Bausatz von Klemmkörpern gemäß Patentanspruch 5.

Im folgenden wird die Erfindung anhand eines Ausführungsbeispiels im Zusammenhang mit der Zeichnung näher erläutert.

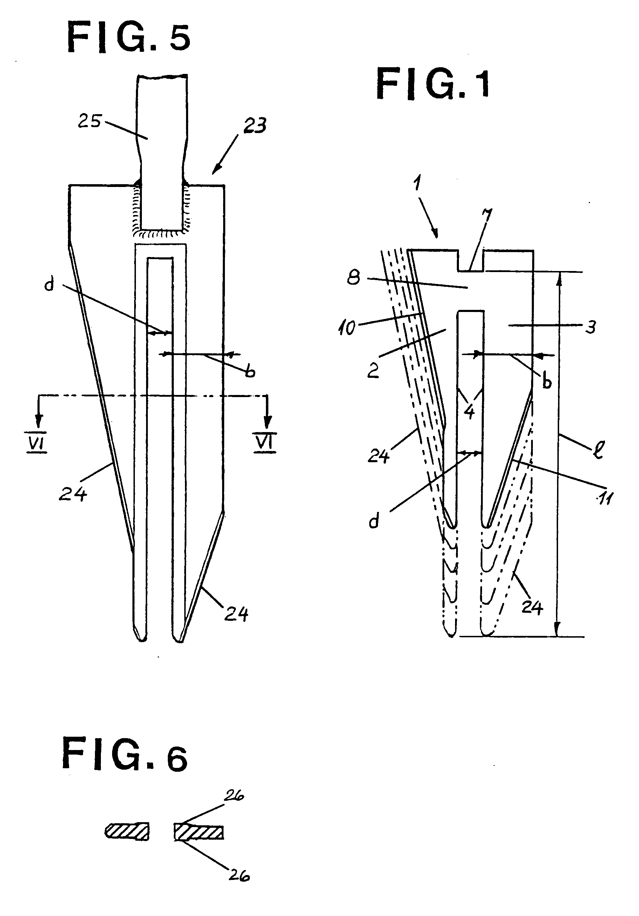

- Fig. 1: zeigt ein Ausführungsbeispiel für einen Klemmkörper und angedeutet die in distaler Richtung versetzten Konturen weiterer Klemmkörper;
- Fig. 2: ist eine Aufsicht auf eine Blattseite eines nur im proximalen Bereich dargestellten Schaftes für eine Hüftgelenksprothese;
- Fig. 3: ist eine Ansicht der Fig. 2 von links;
- Fig. 4: ist eine Aufsicht auf Fig. 2 von oben.
- Fig. 5: zeigt schematisch eine Einschlagschablone mit gleicher Kontur wie die Klemmkörper; und
- Fig. 6: zeigt schematisch den Querschnitt einer Einschlagschablone nach Fig. 5.

Der im Beispiel gezeigte Klemmkörper 1, der aus einem etwa drei Millimeter (mm) dickem Blech besteht, ist in seiner Grundform H-förmig; er weist eine unsymmetrische Form auf, wobei seine nach unten weisenden freien Schenkel 2 und 3 sowohl in ihrer Breite b als auch in ihrer Form verschieden sind. Beide Schenkel 2, 3 sind darüberhinaus zu ihrem freien Ende hin angeschrägt, um das Eindringen in den Knochen zu erleichtern und das Abstützen in der Korticalis sicherzustellen.

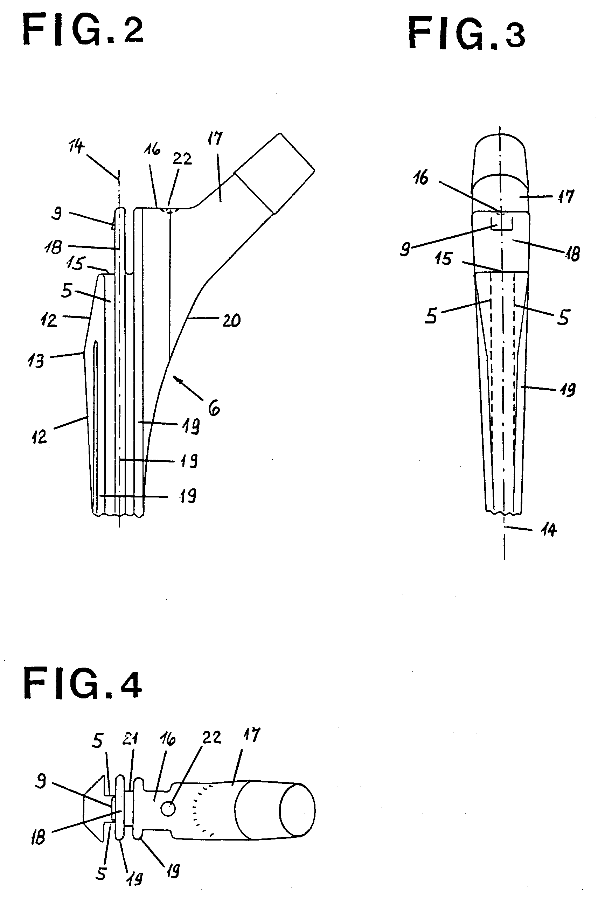

Die "inneren", zueinander parallelen Kanten 4 der Schenkel 2, 3 haben einen Abstand d voneinander, der der "Dicke" des Schaftes 6 im Bereich der Führungen 5 (Fig. 4) entspricht. Die obere Kante 7 des Quersteges 8 der H-Form bildet bei dem gezeigten Klemmkörper 1 eine Vertiefung, in die eine Nase 9 (Fig. 2) an einem blattfederartig federnden Sicherungselement 18 (Fig. 2) des Schaftes 6, das später beschrieben wird, einrasten kann. Die Anschrägung 10 des Schenkels 2 und die Anschrägung 11 des Schenkels 3 in der sagittalen Ebene sind unterschiedlich, ihre relative Lage zueinander bestimmt ein Kontur 24, die sich bei allen Klemmkörpern 1 der gleichen Breite b mit unterschiedlichem Längsversatz l wiederholt, damit innerhalb dieser Gruppe eine Einschlagschablone 23 mit der gleichen Kontur 24 wie die der Klemmkörper 1 verwendbar ist.

Das nur im proximalen Bereich gezeigte Blatt des Schaftes 6, das sich von distal nach proximal zunächst allseitig - wie Fig. 2 und 3 erkennen lassen - konisch erweitert, hat eine laterale Schmalseite 12, die ab einer Unstetigkeitsstelle 13 in Richtung auf die Längsmittelachse 14 des Schaftes 6 zu verläuft. Sie endet in einem Absatz 15 unterhalb einer senkrecht zur Längsmittelachse 14 verlaufenden Schulter 16 am proximalen Schaftende, die in einen Prothesenhals 17 übergeht, der seinerseits den nicht gezeigten Gelenkkopf der Prothese aufnimmt. Die mediale Schmalseite 20 des Schaftes 6 verläuft aus ihrer konischen Erweiterung heraus im gezeigten Bereich bogenförmig und endet ebenfalls im Prothesenhals 17.

Die Blattseiten des sich nach proximal konisch erweiternden Schaftes 6 sind mit in Richtung der Längsmittelachse 14 verlaufenden Rippen 19 versehen; mindestens eine der einander gegenüberliegenden Talsohlen 21 zwischen je zwei dieser Rippen 19 ist als Führung 5 konstanter Dicke d für die Schenkel 2, 3 des Klemmkörpers 1 ausgebildet, wie besonders aus Fig. 3 zu entnehmen ist.

Oberhalb des Absatzes 15 ist eine der an die Führung 5 angrenzenden Rippen 19 in Richtung der Längsmittelachse 14 zu einem blattfederartig federnden Sicherungselement 18 verlängert, das sich in seiner Höhe bis zur Schulter 16 erstreckt. Es trägt auf seiner der Führung 5 zugewandten Federblattfläche die Nase 9, die bei eingeschlagenem Klemmkörper 1 in eine Vertiefung einrastet, um eine unbeabsichtigte Lockerung des im Knochen eingekeilten Klemmkörpers 1 zu verhindern. Im Falle einer Reoperation lässt sich das Element 18 jedoch elastisch verbiegen, wodurch die Nase 9 ausrasten und der Klemmkörper 1, sowie nach ihm der Schaft 6, aus dem Knochen herausgezogen werden können.

Mit 22 eine Ausnehmung am Schaft 6 bezeichnet, die zum Ansetzen von Einschlagelementen für den Schaft 6 dient.

Figur 5 und 6 zeigen eine Einschlagschablone 23, welche entlang der Führung 5 am bereits eingeschlagenen Schaft 6 in den Knochenhohlraum eingefahren wird bis sie fest auf der Korticalis aufsitzt. Sie besitzt Führungsflächen 26, die im Bereich der Führung der Blechdicke der Klemmkörper 1 entsprechen, während im restlichen Bereich die Dicke reduziert ist. Die beiden Schenkel der Einschlagschablone 23 weisen die gleiche Kontur 24 wie die Klemmkörper 1 mit ihren Anschrägungen 10, 11 auf. Sie sind gegen distal zugeschärft, um die Verdrängung von Knochengewebe zu erleichtern. Aufgrund der erreichten Eindringtiefe der Einschlagschablone 23 wird ein Klemmkörper 1 ausgelesen, dessen Kontur 24 die passende Lage in Richtung der Längsmittelachse 14 aufweist, bei der das Sicherungselement 18 am Klemmkörper 1 einrastet und bei der eine Klemmung gegen das Knochengewebe stattfindet. Nach dem Ziehen der Einschlagschablone 23 wird der so ausgelesene Klemmkörper 1 eingeschlagen bis eine Verklinkung mit dem Sicherungselement stattgefunden hat. Da die Blechdicke der Klemmkörper 1 im verdrängenden Bereich grösser als die der Einschlagschablone 23 ist, wird der Klemmkörper 1 sicher verkeilt.

Die Kraftübertragung auf die Einschlagschablone 23 erfolgt durch einen angeschweissten Stössel 25. Der Führungsschlitz mit dem Abstand d ist soweit nach oben gezogen, dass beim Einschlagen der Einschlagprothese keine Beeinträchtigung durch die Nase 9 erfolgt.

Zur Operationsplanung werden Röntgenschablonen der verschieden Schaftgrössen und der Einschlagschablonen 23 mit unterschiedlicher Breite b verwendet.

## Patentansprüche

1. Gerader, blattartiger Schaft (6) für eine Endoprothese, insbesondere eine Hüftgelenksprothese, dessen Blattseiten (21) sich vom distalen, freien Ende aus zunächst symmetrisch zu einer Langsmittelachse (14) konisch erweitern, ehe die laterale Schmalseite (12) parallel zur Längsmittelachse (14) oder auf sie zu verläuft, während die mediale Schmalseite (20) in einem Bogen geführt ist, wobei im proximalen Bereich des Schaftes Führungen (5) vorgesehen sind, in die mindestens ein Klemmkörper (1) einschlagbar ist, dadurch gekennzeichnet, dass der Klemmkörper (1) ein U-förmiges Blech ist, dessen freie Schenkel (2, 3) zu ihrem Ende hin abgeschrägt sind, und dass ferner parallel zu den Führungen (5) des Schaftes (6) ein blattfederartig federndes Sicherungselement (18) vorgesehen ist, das auf der den Führungen (5) zugewandten Blattfederseite einen nasenartigen Vorsprung (9) trägt, der in eine Vertiefung (7) des eingeschlagenen Klemmkörpers (1) einrastet.

2. Schaft nach Anspruch 1, dadurch gekennzeichnet, dass die Führungen (5) in den Blattseiten (21) parallel zur Längsmittelachse (14) verlaufen.

3. Schaft nach Anspruch 1 oder 2, dadurch gekennzeichnet, dass die U-Form des Klemmkörpers (1) zu einer H-Form erweitert ist, deren Querbalken (8) eine Kante (7) zum Einrasten des nasenartigen Vorsprunges (9) bildet.

4. Schaft nach einem der Ansprüche 1 bis 3, dadurch gekennzeichnet, dass der Schaftkörper (6) symmetrisch zu der zu seinen Blattseiten (21) parallel verlaufenden Mittelebene ausgebildet ist, während der senkrecht zu dieser Ebene verlaufende, U-förmige Teil vom Klemmkörper (1) eine unsymmetrische Form hat, wobei seine Schenkel (2, 3) ungleich breit und ungleich angeschrägt sind.

5. Bausatz von Klemmkörpern (1) nach einem der Ansprüche 1 bis 4 zum Verankern eines blattartigen Schafts, dadurch gekennzeichnet, dass die Klemmkörper sich voneinander unterscheiden, indem die anterior und posterior tragenden Teile der Schenkel (2, 3) mit ihrer Kontur (24) durch stufenweise Parallelverschiebung in der Längsmittelachse (14) in unterschiedlichem Längsversatz (l) auseinander hervorgehen.

6. Bausatz nach Anspruch 5, dadurch gekennzeichnet, dass er mindestens eine Einschlagschablone (23) mit gleicher Kontur (24) aufweist, um aufgrund einer im Knochen erreichbaren Eindringtiefe den Klemmkörper mit dem passenden Längsversatz (l) seiner Kontur (24) zu bestimmen.

## Claims

1. A straight bladelike shank (6) for an endoprosthesis, in particular a hipjoint prosthesis, the sides (21) of the blade of which first of all widen out symmetrically with respect to a longitudinal centreline (14) until the lateral narrow side (12) runs in parallel with the longitudinal centreline (14) or in towards it, whilst the medial narrow side (20) is led in an arc, and in which guides (5) into which at least one clamp body (1) may be driven are provided in the proximal region of the shank, characterized in that the clamp body (1) is a U-shaped piece of sheetmetal of which the free arms (2, 3) are bevelled towards their ends, and further that a springy locking member (18) like a leaf spring is provided on the shank (6) in parallel with the guides (5) and carries on the leafspring side next the guides (5) a noselike projection (9) which snaps into a depression (7) in the clamp body (1) when the latter has been driven in.

2. A shank as in Claim 1, characterized in that the guides (5) in the sides (21) of the blade run in parallel with its centreline (14).

3. A shank as in Claim 1 or 2, characterized in that the U-shape of the clamp body (1) is widened into the shape of an H of which the cross-bar (8) forms an edge (7) for snapping in the noselike projection (9).

4. A shank as in one of the Claims 1 to 3, characterized in that the shank body (6) is made symmetrical about the central plane running in parallel with the sides (21) of its blade, whilst the U-shaped part of the clamp body (1) running perpendicular to this plane has an asymmetrical shape, its arms (2, 3) being of unequal width and unequally bevelled.

5. A constructional kit of clamp bodies (1) as in one of the Claims 1 to 4, for anchoring a bladelike shank, characterized in that the clamp bodies differ from one another in that the anterior and posterior carrying parts of the arms (2, 3) follow from one another by parallel shifting of their contour (24) by different longitudinal offsets (*l*) in steps along the longitudinal centreline (14).

6. A constructional kit as in Claim 5, characterized in that it exhibits at least one drive-in template (23) of the same contour (24), in order on the basis of a depth of penetration which may be reached in the bone to determine the clamp body which has the appropriate longitudinal offset (*l*) of its contour (24).

## Revendications

1. Tige droite (6), en forme de lame, pour une endoprothèse, en particulier une prothèse d'articulation de la hanche, dont les faces de lame (21) vont en s'agrandissant de façon conique, depuis une extrémité libre distale, en évoluant d'abord symétriquement par rapport à un axe médian longitudinal (14), avant que le côté étroit (12) latéral s'étende parallèlement à l'axe médian longitudinal (14) ou en direction de lui, tandis que le côté étroit (20) médial évolue en suivant un arc, dans la zone proximale de la tige étant prévus des guidages (5) dans lesquels au moins un corps de serrage (1) peut être inséré par martelage, caractérisée en ce que le corps de serrage (1) est une tôle en forme de U dont les branches libres (2, 3) sont biseautées à leur extrémité, et en ce que, en outre, parallèlement aux guidages (5) de la tige (6) est prévu un élément de sécurité (18) élastique du genre d'un ressort à lame, qui porte, sur la face du ressort à lame tournée vers les guidages (5), une saillie (9) du genre d'un ergot qui s'encliquette dans une cavité (7) du corps de serrage (1) ayant été introduit par martelage.

2. Tige selon la revendication 1, caractérisée en ce que les guidages (5) s'étendent, dans les faces de lame (21), parallèlement à l'axe médian longitudinal (14).

3. Tige selon la revendication 1 ou 2, caractérisée en ce que la forme en U du corps de serrage (1) est élargie en une forme en H dont le tronçon transversal (8) constitue une arête (7) utilisée pour l'encliquetage de la saillie (9) en ergot.

4. Tige selon l'une des revendications 1 à 3, caractérisée en ce que le corps de tige (6) est réalisé symétriquement par rapport au plan médian courant parallèlement par rapport à ses faces de lame (21), tandis que la partie en forme de U, courant perpendiculairement par rapport à ce plan, du corps de serrage (1), a une forme asymétrique, ses branches (2, 3) étant de largeurs différentes et ayant un biseautage différent.

5. Jeu de construction constitué de corps de serrage (1), selon l'une des revendications 1 à 4, pour ancrage d'une tige du genre d'une lame, caractérisé en ce que les corps de serrage se distinguent les uns des autres par le fait que les parties porteuses antérieure et postérieure des branches (2, 3) résultent les unes des autres, par leur contour (24), par déplacement parallèle, fait par degrés, dans l'axe médian longitudinal (14), avec un déplacement longitudinal (1) différent.

6. Jeu de construction selon la revendication 5, caractérisé en ce qu'il présente au moins un gabarit de martelage (23) ayant un contour (24) identique, pour, sur la base d'une profondeur de pénétration pouvant être atteinte dans l'os, déterminer quel est le corps de serrage qui présente un déplacement longitudinal (1) adapté de son contour (24).
